# EUROPEAN PATENT APPLICATION

(11) **EP 2 210 603 A1**
(43) Date of publication of application: **28.07.2010**
(21) Application number: 08835097.0
(22) Date of filing: 02.10.2008
(51) Int. Cl.: A61K 31/277, A61P 3/10, A61P 9/04, A61P 11/00, A61P 13/12, A61P 25/00, A61P 25/28, A61P 29/00, A61P 31/06, A61P 31/08, A61P 43/00

(54) **PHARMACEUTICAL COMPOSITION FOR INHIBITING THE ACCUMULATION OF AMYLOID- B PROTEIN**

(30) Priority: 03.10.2007 JP 2007259565
(71) Applicant: Sumitomo Chemical Company, Limited, Tokyo 104-8260 (JP)
(72) Inventor: MOTONAGA, Kozo, Ibaraki-shi Osaka 567-0841 (JP); SAITO, Koichi, Takarazuka-shi Hyogo 665-0847 (JP)
(74) Representative: Duckworth, Timothy John
(86) International application number: PCT/JP2008/068321
(87) International publication number: WO 2009/044930

(57) **Abstract**

The present invention provides: a pharmaceutical composition for inhibiting amyloid-β protein accumulation comprising a compound of the formula (I) or a pharmaceutically acceptable salt thereof; a method for inhibiting amyloid-β protein accumulation, comprising a step of administering an effective amount of the compound of the formula (I) or a pharmaceutically acceptable salt thereof to a mammal which may be diagnosed with an amyloid-β protein-related disease; and so on.

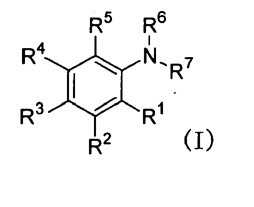

## Description

### Technical Field

The present invention relates to a pharmaceutical composition that inhibits amyloid-β protein accumulation, and so on.

### Background Art

An amyloid-β protein (hereinafter, sometimes referred to as Aβ) is an insoluble protein consisting of approximately 40 amino acid residues, and is generated by cleavage of an amyloid-β precursor protein by β-secretase and γ-secretase. The β-secretase has been identified as an aspartic protease (Neuron, 27, 419-422, 2000). It has been revealed that a gene responsible for familial Alzheimer's disease (FAD) and presenilin or a complex containing presenilin are involved in expression of a γ-secretase activity (Neuron, 27, 419-422, 2000).

It has been known that accumulation of the Aβ in each organ and tissue in a living body causes functional impairment in the organ and tissue. Diseases in which each organ and tissue is impaired by accumulation of the Aβ are collectively called Aβ-related diseases (amyloidosis).

### Disclosure of the Invention

Based on the foregoing findings, a compound that inhibits Aβ accumulation is useful as a medicine capable of treating or preventing an Aβ-related disease. An object of the present invention is to provide a compound that inhibits Aβ accumulation.

The present invention includes the following inventions.

### [Invention 1]

A pharmaceutical composition for inhibiting amyloid-β protein accumulation comprising a compound of formula (I): 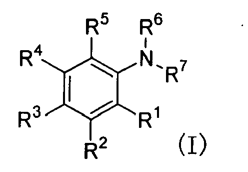 or a pharmaceutically acceptable salt thereof,
in the formula,
R¹ is H, cyano, nitro, halogen, C1-C12 haloalkyl, C1-C12 alkyl, C2-C12 alkenyl, C2-C12 alkynyl, hydroxy, C1-C12 alkoxy, C1-C12 haloalkoxy, -OC(O)R⁸ or aryl;
R² is H, cyano, nitro, halogen, C1-C12 haloalkyl, C1-C12 alkyl, C2-C12 alkenyl, C2-C12 alkynyl, hydroxy, C1-C12 alkoxy, C1-C12 haloalkoxy, -OC(O)R⁸ or aryl;
R³ is cyano, nitro, halogen, C1-C12 haloalkyl, C1-C12 heterocyclyl, hydroxy, C1-C12 alkoxy, C1-C12 haloalkoxy, -OC (O)R⁸, -C(O)OR⁸', CONHR⁸', -C(O)R⁸, -S(O)nR⁸', -SO₂NR⁸'R⁸", -NHC(O)R⁸ or -NHSO₂R⁸, wherein n is 0, 1 or 2;
R⁴ is H, cyano, nitro, halogen, C1-C12 haloalkyl, C1-C12 alkyl, C2-C12 alkenyl, C2-C12 alkynyl, hydroxy, C1-C12 alkoxy, C1-C12 haloalkoxy, -OC(O)R⁸ or aryl;
R⁵ is H, cyano, nitro, halogen, C1-C12 haloalkyl, C1-C12 alkyl, C2-C12 alkenyl, C2-C12 alkynyl, hydroxy, C1-C12 alkoxy, C1-C12 haloalkoxy, -OC(O)R⁸ or aryl;
herein, at least one of R¹ , R², R⁴ and R⁵ is not H;
each of R⁶ and R⁷ independently are selected from H or -(R^{a})x-R⁹;
R^{a} is a C₁-C₈alkylene chain, where x is 0 or 1;
each R⁸ independently is H, C1-C12 alkyl, C2-C12 alkenyl, C2-C12 alkynyl, C3-C12 cycloalkyl, C2-C12 heterocyclyl, aryl, aryl(C1-C12)alkyl, heteroaryl or heteroaryl(C1-C12)alkyl;
R⁸' and R⁸" each independently are H, C1-C12 alkyl, C3-C12 alkenyl, C3-C12 alkynyl, C3-C12 cycloalkyl, C3-C12 heterocyclyl, aryl, aryl(C1-C12)alkyl, heteroaryl or heteroaryl(C1-C12)alkyl;
R⁹ is C1-C12 alkyl, C1-C12 haloalkyl, C2-C12 hydroxyalkyl, C3-C12 alkenyl, C3-C12 alkynyl, C1-C12 alkoxy, C1-C12 alkylthio, C2-C12 haloalkoxy, C3-C12 cycloalkyl, formyl, azide or -NR¹⁰R¹¹;
R¹⁰ and R¹¹ each independently are H, C1-C12 alkyl, -C(O)H, -C(O)R¹², -C(O)OR¹² or -SO₂R¹²; and
R¹² is C1-C12 alkyl;
provided that when R⁶ is methoxy or methylthio, R⁷ simultaneously is not methoxy or methylthio, and when R⁹ is trifluoromethyl or azide, x is not 0, and when R⁹ is azide, R^{a} is not methylene.

### [Invention 2]

An amyloid-β protein accumulation-inhibitory agent for inhibiting amyloid-β protein accumulation comprising the compound of formula (I) described in Invention 1 or a pharmaceutically acceptable salt thereof.

### [Invention 3]

Use of the compound of formula (I) described in Invention 1 or a pharmaceutically acceptable salt thereof for inhibiting amyloid-β protein accumulation.

### [Invention 4]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease.

### [Invention 5]

A pharmaceutical composition for inhibiting amyloid-β protein accumulation comprising the compound of formula (I) described in Invention 1 or a pharmaceutically acceptable salt thereof, wherein at least one of R¹ and R⁵ is H.

### [Invention 6]

An amyloid-β protein accumulation-inhibitory agent for inhibiting amyloid-β protein accumulation comprising the compound of formula (I) described in Invention 1 or a pharmaceutically acceptable salt thereof, wherein at least one of R¹ and R⁵ is H.

### [Invention 7]

Use of the compound of formula (I) described in Invention 1 or a pharmaceutically acceptable salt thereof for inhibiting amyloid-β protein accumulation, wherein at least one of R¹and R⁵ is H.

### [Invention 8]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein at least one of R¹ and R⁵ is H.

### [Invention 9]

A pharmaceutical composition for inhibiting amyloid-β protein accumulation comprising the compound of formula (I) described in Invention 1 or a pharmaceutically acceptable salt thereof, wherein at least one of R² and R⁴ is H.

### [Invention 10]

An amyloid-β protein accumulation-inhibitory agent for inhibiting amyloid-β protein accumulation comprising the compound of formula (I) described in Invention 1 or a pharmaceutically acceptable salt thereof, wherein at least one of R² and R⁴ is H.

### [Invention 11]

Use of the compound of formula (I) described in Invention 1 or a pharmaceutically acceptable salt thereof for inhibiting amyloid-β protein accumulation, wherein at least one of R² and R⁴ is H.

### [Invention 12]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein at least one of R² and R⁴ is H.

### [Invention 13]

A pharmaceutical composition for inhibiting amyloid-β protein accumulation comprising the compound of formula (I) described in Invention 1 or a pharmaceutically acceptable salt thereof, wherein C1-C12 alkyl is C1-C6 alkyl.

### [Invention 14]

An amyloid-β protein accumulation-inhibitory agent for inhibiting amyloid-β protein accumulation comprising the compound of formula (I) described in Invention 1 or a pharmaceutically acceptable salt thereof, wherein C1-C12 alkyl is C1-C6 alkyl.

### [Invention 15]

Use of the compound of formula (I) described in Invention 1 or a pharmaceutically acceptable salt thereof for inhibiting amyloid-β protein accumulation, wherein C1-C12 alkyl is C1-C6 alkyl.

### [Invention 16]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein C1-C12 alkyl is C1-C6 alkyl.

### [Invention 17]

A pharmaceutical composition for inhibiting amyloid-β protein accumulation comprising the compound of formula (I) described in Invention 1 or a pharmaceutically acceptable salt thereof, wherein C1-C12 alkoxy is C1-C6 alkoxy.

### [Invention 18]

An amyloid-β protein accumulation-inhibitory agent for inhibiting amyloid-β protein accumulation comprising the compound of formula (I) described in Invention 1 or a pharmaceutically acceptable salt thereof, wherein C1-C12 alkoxy is C1-C6 alkoxy.

### [Invention 19]

Use of the compound of formula (I) described in Invention 1 or a pharmaceutically acceptable salt thereof for inhibiting amyloid-β protein accumulation, wherein C1-C12 alkoxy is C1-C6 alkoxy.

### [Invention 20]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein C1-C12 alkoxy is C1-C6 alkoxy.

### [Invention 21]

A pharmaceutical composition for inhibiting amyloid-β protein accumulation comprising the compound of formula (I) described in Invention 1 or a pharmaceutically acceptable salt thereof, wherein C1-C12 haloalkyl is C1-C6 haloalkyl.

### [Invention 22]

An amyloid-β protein accumulation-inhibitory agent for inhibiting amyloid-β protein accumulation comprising the compound of formula (I) described in Invention 1 or a pharmaceutically acceptable salt thereof, wherein C1-C12 haloalkyl is C1-C6 haloalkyl.

### [Invention 23]

Use of the compound of formula (I) described in Invention 1 or a pharmaceutically acceptable salt thereof for inhibiting amyloid-β protein accumulation, wherein C1-C12 haloalkyl is C1-C6 haloalkyl.

### [Invention 24]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein C1-C12 haloalkyl is C1-C6 haloalkyl.

### [Invention 25]

A pharmaceutical composition for inhibiting amyloid-β protein accumulation comprising the compound of formula (I) described in Invention 1 or a pharmaceutically acceptable salt thereof, wherein C2-C12 alkenyl of R¹, R², R⁴, R⁵ and R⁸ is C2-C6 alkenyl, and wherein C3-C12 alkenyl of R⁸', R⁸" and R⁹ is C3-C6 alkenyl.

### [Invention 26]

An amyloid-β protein accumulation-inhibitory agent for inhibiting amyloid-β protein accumulation comprising the compound of formula (I) described in Invention 1 or a pharmaceutically acceptable salt thereof, wherein C2-C12 alkenyl of R¹ , R², R⁴, R⁵ and R⁸ is C2-C6 alkenyl, and wherein C3-C12 alkenyl of R⁸', R⁸" and R⁹ is C3-C6 alkenyl.

### [Invention 27]

Use of the compound of formula (I) described in Invention 1 or a pharmaceutically acceptable salt thereof for inhibiting amyloid-β protein accumulation, wherein C2-C12 alkenyl of R¹ , R², R⁴, R⁵ and R⁸ is C2-C6 alkenyl, and wherein C3-C12 alkenyl of R⁸', R⁸", and R⁹ is C3-C6 alkenyl.

### [Invention 28]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein C2-C12 alkenyl of R¹, R² , R⁴, R⁵ and R⁸ is C2-C6 alkenyl, and wherein C3-C12 alkenyl of R⁸', R⁸" and R⁹ is C3-C6 alkenyl.

### [Invention 29]

A pharmaceutical composition for inhibiting amyloid-β protein accumulation comprisinig the compound of formula (I) described in Invention 1 or a pharmaceutically acceptable salt thereof, wherein C2-C12 alkynyl of R¹ , R² R⁴, R⁵ and R⁸ is C2-C6 alkynyl, and wherein C3-C12 alkynyl of R⁸', R⁸" and R⁹ is C3-C6 alkynyl.

### [Invention 30]

An amyloid-β protein accumulation-inhibitory agent for inhibiting amyloid-β protein accumulation comprising the compound of formula (I) described in Invention 1 or a pharmaceutically acceptable salt thereof, wherein C2-C12 alkynyl of R¹, R², R⁴, R⁵ and R⁸ is C2-C6 alkynyl, and wherein C3-C12 alkynyl of R⁸', R⁸" and R⁹ is C3-C6 alkynyl.

### [Invention 31]

Use of the compound of formula (I) described in Invention 1 or a pharmaceutically acceptable salt thereof for inhibiting amyloid-β protein accumulation, wherein C2-C12 alkynyl of R¹, R², R⁴, R⁵ and R⁸ is C2-C6 alkynyl, and wherein C3-C12 alkynyl of R⁸', R⁸'_{,} and R⁹ is C3-C6 alkynyl.

### [Invention 32]

A method for inhibiting-amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein C2-C12 alkynyl of R¹ , R² , R⁴ , R⁵ and R⁸ is C2-C6 alkynyl, and wherein C3-C12 alkynyl of R⁸', R⁸'', and R⁹ is C3-C6 alkynyl.

### [Invention 33]

A pharmaceutical composition for inhibiting amyloid-β protein accumulation comprising the compound of formula (I) described in Invention 1 or a pharmaceutically acceptable salt thereof, wherein C3-C12 cycloalkyl is C3-C6 cycloalkyl.

### [Invention 34]

An amyloid-β protein accumulation-inhibitory agent for inhibiting amyloid-β protein accumulation comprising the compound of formula (I) described in Invention 1 or a pharmaceutically acceptable salt thereof, wherein C3-C12 cycloalkyl is C3-C6 cycloalkyl.

### [Invention 35]

Use of the compound of formula (I) described in Invention 1 or a pharmaceutically acceptable salt thereof for inhibiting amyloid-β protein accumulation, wherein C3-C12 cycloalkyl is C3-C6 cycloalkyl.

### [Invention 36]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein C3-C12 cycloalkyl is C3-C6 cycloalkyl.

### [Invention 37]

A pharmaceutical composition for inhibiting amyloid-β protein accumulation comprising the compound of formula (I) described in Invention 1 or a pharmaceutically acceptable salt thereof, wherein R¹ or R⁵ is nitro, C1-C6 alkyl, C1-C6 haloalkyl or halogen.

### [Invention 38]

An amyloid-β protein accumulation-inhibitory agent for inhibiting amyloid-β protein accumulation comprising the compound of formula (I) described in Invention 1 or a pharmaceutically acceptable salt thereof, wherein R¹ or R⁵ is nitro, C1-C6 alkyl, C1-C6 haloalkyl or halogen.

### [Invention 39]

Use of the compound of formula (I) described in Invention 1 or a pharmaceutically acceptable salt thereof for inhibiting amyloid-β protein accumulation, wherein R¹ or R⁵ is nitro, C1-C6 alkyl, C1-C6 haloalkyl or halogen.

### [Invention 40]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein R¹ or R⁵ is nitro, C1-C6 alkyl, C1-C6 haloalkyl or halogen.

### [Invention 41]

A pharmaceutical composition for inhibiting amyloid-β protein accumulation containing the compound of formula (I) described in Invention 1 or a pharmaceutically acceptable salt thereof, wherein R² or R⁴ is nitro, cyano, C1-C6 alkyl, C1-C6 haloalkyl, halogen or hydroxy.

### [Invention 42]

An amyloid-β protein accumulation-inhibitory agent for inhibiting amyloid-β protein accumulation comprising the compound of formula (I) described in Invention 1 or a pharmaceutically acceptable salt thereof, wherein R² or R⁴ is nitro, cyano, C1-C6 alkyl, C1-C6 haloalkyl, halogen or hydroxy.

### [Invention 43]

Use of the compound of formula (I) described in Invention 1 or a pharmaceutically acceptable salt thereof for inhibiting amyloid-β protein accumulation, wherein R² or R⁴ is nitro, cyano, C1-C6 alkyl, C1-C6 haloalkyl, halogen or hydroxy.

### [Invention 44]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein R² or R⁴ is nitro, cyano, C1-C6 alkyl, C1-C6 haloalkyl, halogen or hydroxy.

### [Invention 45]

A pharmaceutical composition for inhibiting amyloid-β protein accumulation comprising the compound of formula (I) described in Invention 1 or a pharmaceutically acceptable salt thereof, wherein R³ is nitro, cyano or halogen.

### [Invention 46]

An amyloid-β protein accumulation-inhibitory agent for inhibiting amyloid-β protein accumulation comprising the compound of formula (I) described in Invention 1 or a pharmaceutically acceptable salt thereof, wherein R³ is nitro, cyano or halogen.

### [Invention 47]

Use of the compound of formula (I) described in Invention 1 or a pharmaceutically acceptable salt thereof for inhibiting amyloid-β protein accumulation, wherein R³ is nitro, cyano or halogen.

### [Invention 48]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein R³ is nitro, cyano or halogen.

### [Invention 49]

A pharmaceutical composition for inhibiting amyloid-β protein accumulation comprising the compound of formula (I) described in Invention 1 or a pharmaceutically acceptable salt thereof, wherein R⁹ is C1-C6 alkyl, C3-C6 alkenyl or C2-C6 hydroxyalkyl.

### [Invention 50]

An amyloid-β protein accumulation-inhibitory agent for inhibiting amyloid-β protein accumulation comprising the compound of formula (I) described in Invention 1 or a pharmaceutically acceptable salt thereof, wherein R⁹ is C1-C6 alkyl, C3-C6 alkenyl or C2-C6 hydroxyalkyl.

### [Invention 51]

Use of the compound of formula (I) described in Invention 1 or a pharmaceutically acceptable salt thereof for inhibiting amyloid-β protein accumulation, wherein R⁹ is C1-C6 alkyl, C3-C6 alkenyl or C2-C6 hydroxyalkyl.

### [Invention 52]

A method for inhibiting amyloid-β protein accumulation, comprising a step of administering an effective amount of the compound of formula (I) described in Invention 1 or a pharmaceutically acceptable salt thereof to a mammal which may be diagnosed with an amyloid-β protein-related disease, wherein R⁹ is C1-C6 alkyl, C3-C6 alkenyl or C2-C6 hydroxyalkyl.

### Brief Description of the Drawings

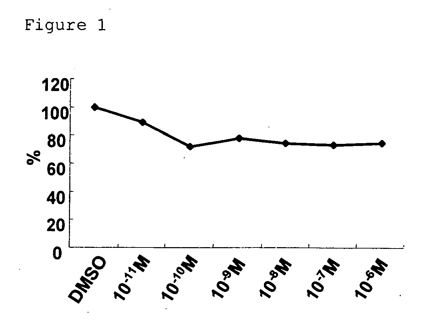

Figure 1 is a diagram showing results of measuring fluctuation caused by a test substance of the formula (II) in the amount of Aβ accumulation in cultured nerve cells (CHP212 cells).

In the diagram, each column represents, starting from the left, a result of measurement obtained in a control well to which only a solvent (DMSO) has been added, in a well to which the test substance has been added so that its final concentration can be 10⁻¹¹ M (10⁻¹¹ M), in a well to which the test substance has been added so that its final concentration can be 10⁻¹⁰ M (10⁻¹⁰ M), in a well to which the test substance A has been added so that its final concentration can be 10⁻⁹ M (10⁻⁹ M), in a well to which the test substance has been added so that its final concentration can be 10⁻⁸ M (10⁻⁸ M) , in a well to which the test substance has been added so that its final concentration can be 10⁻⁷ M (10⁻⁷ M), and in a well to which the test substance has been added so that its final concentration can be 10⁻⁶ M (10⁻⁶ M). The results are shown in % by setting a result obtained in a control well to which only a solvent (DMSO) has been added as 100%.

### Best Mode for Carrying Out the Invention

The present invention will be more specifically described in the following.

Compounds contained in the pharmaceutical composition of the present invention (hereinafter, the compounds may be referred to as "the present compounds" and the composition may be referred to as "the present pharmaceutical composition") are described in, for example, J. Comb. Chem. 2007, 9, 107 or National Publication of International Patent Application No. 2007-500245, and thus structures, production methods, etc. of the present compounds are publicly known.

In the present invention, "alkyl" refers to a straight or branched-chain aliphatic saturated hydrocarbon group, and specific examples thereof include methyl, ethyl, propyl, isopropyl, isobutyl, n-butyl, tert-butyl, isopentyl, and n-pentyl.

As used in the present specification, the preferred number of atoms, such as carbon atoms, will be represented by, for example, the phrase "Cₓ-C_{y} alkyl", which refers to an alkyl group, as herein defined, containing the specified number of carbon atoms. Similar terminology will apply for other preferred terms and ranges as well.

In the present invention, "alkenyl" refers to a straight or branched-chain aliphatic unsaturated hydrocarbon group containing one or more carbon-to-carbon double bonds, and specific examples thereof include vinyl, 2-propenyl, and 3-methyl-2-butenyl.

In the present invention, "alkynyl" refers to a straight or branched-chain aliphatic unsaturated hydrocarbon group containng one or more carbon-to-carbon triple bonds, and specific examples thereof include ethynyl and 2-propynyl.

In the present invention, "alkylene" refers to a straight or branched-chain divalent aliphatic saturated hydrocarbon group, and specific examples thereof include methylene (-CH₂-) and ethylene (-CH₂ -CH₂-).

In the present invention, "cycloalkyl" refers to a monocyclic or polycyclic aliphatic saturated hydrocarbon group, and specific examples thereof include cyclopropyl, cyclobutyl, cyclopentyl, cyclohexyl, and cycloheptyl.

In the present invention, "heterocyclyl" refers to a group forming a three to ten-membered heterocyclic ring containing one or more heteroatoms and optionally containing one or more degrees of unsaturation, which may be optionally substituted and does not overlap with heteroaryl. Examples of the heteroatoms include a nitrogen atom, an oxygen atom and a sulfur atom, including N-oxide, sulfur oxide and dioxide, and may be fused to one or more of heteroaryl ring(s), aryl ring(s), or cycloalkyl ring(s). Specific examples of the ring include tetrahydrofuran, pyran, 1,4-dioxane, 1,3-dioxane, piperazine, pyrrolidine, morpholine, tetrahydrothiopyran, and tetrahydrothiophene.

In the present invention, "aryl" refers to a group forming an aromatic hydrocarbon ring, may be optionally substituted and may be fused to one or more of aryl ring(s) or cycloalkyl ring(s). Specific examples of the ring include benzene, anthracene, phenanthrene, naphthalene, and biphenyl.

In the present invention, "heteroaryl" refers to a group forming an aromatic ring containing one or more heteroatoms, and may be optionally substituted. Examples of the heteroatoms include a nitrogen atom, an oxygen atom and a sulfur atom, including N-oxide, sulfur oxide and dioxide, and may be fused to one or more of heteroaryl ring(s), aryl ring(s), or cycloalkyl ring(s). Specific examples of the ring include furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole, and indazole.

Similarly, "heteroarylalkyl" refers to a heteroaryl group attached through an alkylene linker.

In the present invention, "halogen" refers to fluorine, chlorine, bromine or iodine.

In the present invention, "haloalkyl" refers to a branched or straight-chain saturated hydrocarbon group substituted with at least one halogen, and specific examples thereof include trifluoromethyl and 2,2,2-trifluoroethyl.

In the present invention, "hydroxyalkyl" refers to a branched or straight-chain saturated hydrocarbon group substituted with one or more hydroxyl groups, and specific examples thereof include 2-hydroxyethyl and 2,3-dihydroxypropyl.

In the present invention, "alkoxy" refers to a group -OR, where R denotes C1-C12 alkyl.

In the present invention, "alkylthio" refers to a group -SR, where R denotes the same meaning as described above.

In the present invention, "haloalkoxy" refers to a group -OR' and R' refers to haloalkyl as defined above.

As used in the present specification, the phrase "optionally substituted" or variations thereof denote optional (that is, could occur depending on cases) substitution, including multiple degrees of substitution, with one or more substituents.

Exemplary optional substituents include the following:
C1-C6 acyl;
C1-C6 alkyl;
C2-C6 alkenyl;
C2-C6 alkynyl;
C1-C6 alkylsulfonyl;
C1-C6 alkoxy;
cyano;
halogen;
C1-C6 haloalkyl;
hydroxy;
nitro;
aryl, which may be substituted with C1-C6 acyl, C1-C6 alkoxy, C1-C6 alkyl, C2-C6 alkenyl, C2-C6 alkynyl, C1-C6 alkylsulfonyl, cyano, halogen, C1-C6 haloalkyl, hydroxy or nitro;
heteroaryl, which may be substituted with C1-C6 acyl, C1-C6 alkoxy, C1-C6 alkyl, C2-C6 alkenyl, C2-C6 alkynyl, C1-C6 alkylsulfonyl, cyano, halogen, C1-C6 haloalkyl, hydroxy or nitro; arylsulfonyl, which may be substituted with C1-C6 acyl, C1-C6 alkoxy, C1-C6 alkyl, C2-C6 alkenyl, C2-C6 alkynyl, C1-C6 alkylsulfonyl, cyano, halogen, C1-C6 haloalkyl, hydroxy or nitro;
heteroarylsulfonyl, which may be substituted with C1-C6 acyl, C1-C6 alkoxy, C1-C6 alkyl, C2-C6 alkenyl, C2-C6 alkynyl, C1-C6 alkylsulfonyl, cyano, halogen, C1-C6 haloalkyl, hydroxy or nitro; aryloxy, which may be substituted with C1-C6 acyl, C1-C6 alkoxy, C1-C6 alkyl, C2-C6 alkenyl, C2-C6 alkynyl, C1-C6 alkylsulfonyl, cyano, halogen, C1-C6 haloalkyl, hydroxy or nitro; and
NR^{*}R^{**}, wherein R^{*} and R^{**} each independently are selected from H, C1-C6 alkyl, C3-C6 alkenyl, C3-C6 alkynyl, C3-C6 cycloalkyl, C3-C6 heterocyclyl, aryl, aryl(C1-C6)alkyl, heteroaryl, heteroaryl(C1-C6)alkyl, C1-C6 alkylsulfonyl, arylsulfonyl or heteroarylsulfonyl, and wherein each occurrence of such aryl or heteroaryl may be substituted with one or more C1-C6 acyl, C1-C6 alkoxy, C1-C6 alkyl, C2-C6 alkenyl, C2-C6 alkynyl, C1-C6 alkylsulfonyl, cyano, halogen, C1-C6 haloalkyl, hydroxy or nitro, or R^{*} and R^{**} may combine to form a ring, and the ring optionally having additional heteroatoms, optionally having one or more degrees of unsaturation, and optionally being substituted with C1-C6 acyl, C1-C6 alkoxy, C1-C6 alkyl, C2-C6 alkenyl, C2-C6 alkynyl, C1-C6 alkylsulfonyl, cyano, halogen, C1-C6 haloalkyl, hydroxy or nitro.

The compounds of formula (I) may crystallize in two or more forms, a characteristic known as polymorphism, and such polymorphic forms ("polymorphs") are within the scope of formula (I). Polymorphism generally can occur as a response to changes in temperature, pressure, or both. Polymorphism can also result from variations in the crystallization process. Polymorphs can be distinguished by various physical characteristics known in the art such as x-ray diffraction patterns, solubility, and melting point.

Certain of the compounds described herein contain one or more chiral centers, or may otherwise be capable of existing as multiple stereoisomers. The scope of the present invention includes mixtures of stereoisomers as well as purified enantiomers or enantiomerically/diastereomerically enriched mixtures. Also included within the scope of the invention are the individual isomers of the compounds represented by formula (I), as well as any wholly or partially equilibrated mixtures thereof. The present invention also includes the individual isomers of the compounds represented by the formula above as mixtures with isomers thereof in which one or more chiral centers are inverted.

Examples of C1-C12 alkyl of the above described R¹, R², R⁴, R⁵, R⁸, R^{8'}, R^{8"}, R⁹, R¹⁰, R¹¹ and R¹² preferably include C1-C6 alkyl, and more preferably include methyl, ethyl, propyl, and isopropyl.

Examples of C1-C12 alkoxy of the above described R¹, R², R³, R⁴, R⁵ and R⁹ preferably include C1-C6 alkoxy, and more preferably include methoxy and ethoxy.

Examples of C1-C12 haloalkyl of the above described R¹, R², R³, R⁴, R⁵ and R⁹ preferably include C1-C6 haloalkyl, and more preferably include trifluoromethyl, and 2,2,2-trifluoroethyl.

Examples of C2-C12 alkenyl of the above described R¹, R², R⁴, R⁵ and R⁸ preferably include C2-C6 alkenyl, and more preferably include isopropenyl, 3, 3-dimethyl-2-propenyl, and aryl. Examples of C3-C12 alkenyl of R^{8'} , R^{8"} and R⁹ preferably include C3-C6 alkenyl, and more preferably include isopropenyl, 3,3-dimethyl-2-propenyl, and aryl.

Examples of C2-C12 alkynyl of the above described R¹, R², R⁴, R⁵ and R⁸ preferably include C2-C6 alkynyl, and more preferably include propargyl. Examples of C3-C12 alkynyl of R^{8'}, R^{8"} and R⁹ preferably include C3-C6 alkynyl, and more preferably include propargyl.

Examples of C3-C12 cycloalkyl of the above described R⁸, R^{8'}, R^{8"} and R⁹ preferably include C3-C6 cycloalkyl, and more preferably include cyclopropyl, cyclopentyl, and cyclohexyl.

In one embodiment, it is preferable that the above described R³ be nitro, cyano or halogen, and it is more preferable that, simultaneously, at least one or more of R¹, R², R⁴ and R⁵ be C1-C6 haloalkyl, and it is further preferable that, simultaneously, at least one or more of R² and R⁴ be C1-C6 haloalkyl.

In one embodiment, it is preferable that the above described R³ be nitro, cyano or halogen, and it is more preferable that, simultaneously, at least one or more of R¹, R², R⁴ and R⁵ be halogen, and it is further preferable that, simultaneously, at least one or more of R² and R⁴ be halogen, and it is still further preferable that, simultaneously, at least one or more of R² and R⁴ be chlorine.

In one embodiment, it is preferable that the above described R³ be cyano, and it is more preferable that, simultaneously, at least one or more of R¹, R², R⁴ and R⁵ be nitro.

In one embodiment, it is preferable that the above described R³ be nitro, and it is more preferable that, simultaneously, at least one or more of R¹, R², R⁴ and R⁵ are be cyano.

In one embodiment, it is preferable that the above described R³ be cyano, and it is more preferable that, simultaneously, at least one or more of R² and R⁴ be cyano.

Preferred embodiments of the present compound include:
4-[(Cyclopropylmethyl)(propyl)amino]-2-(trifluoromethyl)benzon itrile;
4-[(cyclopropylmethyl)(propyl)amino]-2-nitrobenzonitrile;
4-(diallylamino)-2-(trifluoromethyl)benzonitrile;
5-[allyl(cyclopentyl)amino]-2-nitrobenzonitrile;
4-[butyl(propyl)amino]-2-nitrobenzonitrile;
4-[ethyl(2-methyl-2-propenyl)amino]-2-nitrobenzonitrile;
4-[butyl(ethyl)amino]-2-(trifluoromethyl)benzonitrile;
4-(dipropylamino)-2-(trifluoromethyl)benzonitrile;
N-butyl-N-ethyl-3-methyl-4-nitroaniline;
5-(diallylamino)-2-nitrobenzonitrile;
5-[(cyclopropylmethyl)(propyl)amino]-2-nitrobenzonitrile;
4-(diallylamino)-2-nitrobenzonitrile;
3-methyl-4-nitro-N,N-dipropylaniline;
4-[sec-butyl(propyl)amino]-2-nitrobenzonitrile;
5-[butyl(ethyl)amino]-2-nitrobenzonitrile;
2-chloro-4-[(cyclopropylmethyl)(propyl)amino]benzonitrile;
5-[butyl(propyl)amino]-2-nitrobenzonitrile;
5-[(2-methoxyethyl)((methyl)amino]-2-nitrobenzonitrile;
2-chloro-4-(diallylamino)benzonitrile;
4-[(2-methoxyethyl)(propyl)amino]-2-nitrobenzonitrile;
4-[allyl(cyclopentyl)amino]-2-nitrobenzonitrile;
4-[ethyl(2-methyl-2-propenyl)amino]-2-(trifluoromethyl)benzonitrile;
2-chloro-4-[[2-(dimethylamino)ethyl](methyl)amino]benzonitrile ;
N-(2-methoxyethyl)-N,2-dimethyl-4-nitroaniline;
N-allyl-N-cyclopentyl-4-nitro-3-(trifluoromethyl)aniline;
5-(dipropylamino)-2-nitrophenol;
2-chloro-4-(dipropylamino)benzonitriie;
5-[ethyl(2-methyl-2-propenyl)amino]-2-nitrobenzonitrile;
N,N-diallyl-4-nitro-3-(trifluoromethyl)aniline;
N,N-diallyl-2-methyl-4-nitroaniline;
5-[(cyclopropylmethyl)(propyl)amino]-2-nitrophenol;
N-(2-methoxyethyl)-3-methyl-4-nitro-N-propylaniline;
4-[butyl(propyl)amino]-2-chlorobenzonitrile;
N-butyl-2-chloro-N-methyl-4-nitroaniline;
4-(dipropylamino)-2-nitrobenzonitrile;
2-chloro-4-[(2-methoxyethyl)(propyl)amino]benzonitrile;
N,N-diallyl-3-methyl-4-nitroaniline;
N-(sec-butyl)-4-nitro-N-propyl-3-(trifluoromethyl)aniline;
4-(dipentylamino)-2-nitrobenzonitrile;
N-(cyclopropylmethyl)-3-methyl-4-nitro-N-propylaniline;
5-(dipropylamino)-2-nitrobenzonitrile;
N,N-dibutyl-4-nitro-3-(trifluoromethyl)aniline;
N1-(2-chloro-4-nitrophenyl)-N1,N2,N2-trimethyl-1,2-ethanediami ne;
5-[sec-butyl(propyl)amino]-2-nitrobenzonitrile;
N-ethyl-N-(2-methyl-2-propenyl)-4-nitro-3-(trifluoromethyl)ani line;
N-(2-methoxyethyl)-4-nitro-N-propyl-3-(trifluoromethyl)aniline ;
4-[butyl(ethyl)amino]-2-chlorobenzonitrile;
5-(dibutylamino)-2-nitrobenzonitrile;
4-[butyl(ethyl)amino]-2-nitrobenzonitrile;
4-[(2-methoxyethyl)(propyl)amino]-2-(trifluoromethyl)benzonitr ile;
5-[(2-methoxyethyl)(propyl)amino]-2-nitrobenzonitrile;
4-[allyl(cyclopentyl)amino]-2-(trifluoromethyl)benzonitrile;
4-[butyl(propyl)amino]-2-(trifluoromethyl)benzonitrile;
N,N-dibutyl-3-methyl-4-nitroaniline;
4-[methyl(octyl)amino]-2-nitrobenzonitrile;
4-(dibutylamino)-2-(trifluoromethyl)benzonitrile;
N-(cyclopropylmethyl)-4-nitro-N-propyl-2-(trifluoromethyl)anil ine;
N-allyl-N-cyclohexyl-4-nitro-3-(trifluoromethyl)aniline;
4-[(2-methoxyethyl)(methyl)amino]-3-(trifluoromethyl)benzonitr ile;
4-(diallylamino)-3-nitrobenzonitrile;
N-1-(2-chloro-4-nitrophenyl)-N1,N3,N3-trimethyl-1,3-propanedia mine;
N,N-bis(2-methoxyethyl)-4-nitro-3-(trifluoromethyl)aniline;
4-[sec-butyl(propyl)amino]-2-(trifluoromethyl)benzonitrile;
2-chloro-4-[ethyl(2-methyl-2-propenyl)amino]benzonitrile;
N-cyclohexyl-N-ethyl-4-nitro-3-(trifluoromethyl)aniline;
2-chloro-4-(dibutylamino)benzonitrile;
4-[cyclohexyl(ethyl)amino]-2-nitrobenzonitrile;
4-[bis(2-ethoxyethyl)amino]-3-chlorobenzonitrile;
2-chloro-N-(2-methoxyethyl)-N-methyl-4-nitroaniline;
N-butyl-N-ethyl-4-nitro-3-(trifluoromethyl)aniline;
N-(sec-butyl)-3-methyl-4-nitro-N-propylaniline;
N-(2-methoxyethyl)-N-methyl-4-nitro-2-(trifluoromethyl)aniline ;
4-{bis[3-(dimethylamino)propyl]amino}-2-(trifluoromethyl)benzo nitrile;
N-(cyclopropylmethyl)-4-nitro-N-propyl-3-(trifluoromethyl)aniline;
4-[(methyl)(octyl)amino]-2-(trifluoromethyl)benzonitrile;
4-(propylamino)-2-(trifluoromethyl)benzonitrile;
4-nitro-N-propyl-3-(trifluoromethyl)aniline;
3-{[4-nitro-3-(trifluoromethyl)phenyl]amino}propan-1-ol;
4-[(3-hydroxypropyl)amino]-2-(trifluoromethyl)benzonitrile;
4-{[2-hydroxy-1-(hydroxymethyl)ethyl]amino}-2-(trifluoromethyl )benzonitrile;
4-(dimethylamino)-2-(trifluoromethyl)benzonitrile;
4-(diethylamino)-2-(trifluoromethyl)benzonitrile;
4-[methyl(2-methylpropyl)amino]-2-(trifluoromethyl)benzonitril e;
4-[(cyclopropylmethyl)amino]-2-(trifluoromethyl)benzonitrile;
4-[(cyclopropylmethyl)(3-hydroxypropyl)amino]-2-(trifluorometh yl)benzonitrile;
4-[(cyclopropylmethyl)(2-hydroxyethyl)amino]-2-(trifluoromethy 1)benzonitrile;
N-(cyclopropylmethyl)-4-nitro-3-(trifluoromethyl)aniline;
3-{(cyclopropylmethyl)[4-nitro-3-(trifluoromethyl)phenyl]amino }-1-propanol;
2-{(cyclopropylmethyl)[4-nitro-3-(trifluoromethyl)phenyl]amino }ethanol;
4-[(cyclopropylmethyl)amino]-3-(trifluoromethyl)benzonitrile;
1-[4-[(cyclopropylmethyl)(propyl)amino]-2-(trifluoromethyl)phe nyl]ethanone;
4-[(1-cyclopropylethyl)amino]-2-(trifluoromethyl)benzonitrile;
4-[allyl(1-cyclopropylethyl)amino]-2-(trifluoromethyl)benzonit rile;
4-[(2,2-dimethylpropyl)amino]-2-(trifluoromethyl)benzonitrile;
4-[(2,2-dimethylpropyl)(3-hydroxypropyl)amino]-2-(trifluoromet hyl)benzonitrile;
4-[(2,2-dimethylpropyl)(2-propen-1-yl)amino]-2-(trifluoromethy l)benzonitrile;
4-[(2,3-dihydroxypropyl)(2,2-dimethylpropyl)amino]-2-(trifluor omethyl)benzonitrile;
4-[(2,2-dimethylpropyl)(2-oxoethyl)amino]-2-(trifluoromethyl)b enzonitrile;
4-[(2,2-dimethylpropyl)(2-hydroxyethyl)amino]-2-(trifluorometh yl)benzonitrile;
4-[(2,2-dimethylpropyl)(propyl)amino]-2-(trifluoromethyl)benzo nitrile;
4-[(1,1-dimethylethyl)amino]-2-(trifluoromethyl)benzonitrile;
N-(1,1-dimethylethyl)-4-nitro-3-(trifluoromethyl)aniline;
N-(cyclopropylmethyl)-N-(1,1-dimethylethyl)-4-nitro-3-(trifluo romethyl)aniline;
4-[(1,1-dimethylethyl)(2-propen-1-yl)amino]-2-(trifluoromethyl )benzonitrile;
4-[(1,1-dimethylethyl)(2-oxoethyl)amino]-2-(trifluoromethyl)be nzonitrile;
4-[(1,1-dimethylethyl)(propyl)amino]-2-(trifluoromethyl)benzon itrile;
4-{(3-hydroxypropyl)[(1S)-1-methylpropyl]amino}-2-(trifluoromethyl)benzonitrile;
2-(trifluoromethyl)-4-[(3,3,3-trifluoropropyl)amino]benzonitri le;
4-[bis(2-fluoroethyl)amino]-2-(trifluoromethyl)benzonitrile;
4-[(2,2,2-trifluoroethyl)amino]-2-(trifluoromethyl)benzonitrile;
4-nitro-N-(2,2,2-trifluoroethyl)-3-(trifluoromethyl)aniline
4-[(3-hydroxypropyl)(2,2,2-trifluoroethyl)amino]-2-(trifluorom ethyl)benzonitrile;
4-[(3-hydroxypropyl)(2,2,2-trifluoroethyl)amino]-3-(trifluorom ethyl)benzonitrile;
4-bromo-N,N-bis(2,2,2-trifluoroethyl)-3-(trifluoromethyl)anili ne;
4-[bis(2,2,2-trifluoroethyl)amino]-2-(trifluoromethyl)benzonit rile;
4-[(2,2-difluoroethyl)(2,2,2-trifluoroethyl)amino]-2-(trifluor omethyl)benzonitrile;
bis(2,2,2-trifluoroethyl)[2-(trifluoromethyl)-4-biphenylyl]ami ne;
4-[(2-hydroxyethyl)(2,2,2-trifluoroethyl)amino]-2-(trifluorome thyl)benzonitrile;
4-[[2-(methyloxy)ethyl](2,2,2-trifluoroethyl)amino]-2-(trifluo romethyl)benzonitrile;
4-[[2-(ethyloxy)ethyl](2,2,2-trifluoroethyl)amino]-2-(trifluor omethyl)benzonitrile;
4-((2,2,2-trifluoroethyl){2-[(2,2,2-trifluoroethyl)oxy]ethyl}a mino)-2-(trifluoromethyl)benzonitrile;
4-[methyl(2,2,2-trifluoroethyl)amino]-2-(trifluoromethyl)benzo nitrile;
4-[ethyl(2,2,2-trifluoroethyl)amino]-2-(trifluoromethyl)benzon itrile;
4-[propyl(2,2,2-trifluoroethyl)amino]-2-(trifluoromethyl)benzo nitrile;
4-[butyl(2,2,2-trifluoroethyl)amino]-2-(trifluoromethyl)benzon itrile;
4-[(2-methylprop-2-enyl)(2,2,2-trifluoroethyl)amino]-2-(triflu oromethyl)benzonitrile;
4-[isobutyl(2,2,2-trifluoroethyl)amino]-2-(trifluoromethyl)ben zonitrile;
4-[(3-methylbut-2-enyl)(2,2,2-trifluoroethyl)amino]-2-(trifluo romethyl)benzonitrile;
4-[isopentyl(2,2,2-trifluoroethyl)amino]-2-(trifluoromethyl)be nzonitrile;
4-[prop-2-ynyl(2,2,2-trifluoroethyl)amino]-2-(trifluoromethyl) benzonitrile;
4-[(2-fluoroethyl)(2,2,2-trifluoroethyl)amino]-2-(trifluoromet hyl)benzonitrile;
4-[[2-(methylthio)ethyl](2,2,2-trifluoroethyl)amino]-2-(triflu oromethyl)benzonitrlle;
4-[(2-azidoethyl)(2,2,2-trifluoroethyl)amino]-2-(trifluorometh yl)benzonitrile;
N-{2-[[4-cyano-3-(trifluoromethyl)phenyl](2,2,2-trifluoroethyl )amino]ethyl}acetamide;
methyl 2-[[4-cyano-3-(trifluoromethyl)phenyl](2,2,2-trifluoroethyl)amino]ethylcarbamate;
tert-butyl 2-[[4-cyano-3-(trifluoromethyl)phenyl](2,2,2-trifluoroethyl)amino]ethylcarbamate;
N-{2-[[4-cyano-3-(trifluoromethyl)phenyl](2,2,2-trifluoroethyl )amino]ethyl}methanesulfonamide; and
4-(dipropylamino)phthalonitrile.

The present pharmaceutical composition contains the present compound or a pharmaceutically acceptable salt thereof.

Examples of the pharmaceutically acceptable salt include a salt with a physiologically acceptable acid (an inorganic acid, an organic acid, and so on) or base (an alkaline metal and so on), and a physiologically acceptable acid addition salt is particularly preferred. Examples of such acid addition salt include a salt with an inorganic acid (hydrochloric acid, phosphoric acid, hydrobromic acid, sulfuric acid, and so on) and a salt with an organic acid (acetic acid, formic acid, propionic acid, fumaric acid, maleic acid, succinic acid, tartaric acid, citric acid, malic acid, oxalic acid, benzoic acid, methanesulfonic acid, benzenesulfonic acid, and so on).

The present pharmaceutical composition may contain, for example, a pharmaceutically acceptable carrier, and so on, in addition to the above described present compound or a pharmaceutically acceptable salt thereof.

For the pharmaceutically acceptable carrier, various organic or inorganic carrier substances that are commonly used as general pharmaceutical composition materials, and for example, an excipient, a lubricant, a binder, a disintegrating agent, a solvent, a solubilizer, a suspending agent, an isotonic agent, a buffer, and a soothing agent are used. In addition, additives such as an antiseptic agent, an antioxidant, a coloring agent, and a sweetening agent can also be used, if necessary.

Examples of the excipient include lactose, white sugar, D-mannitol, D-solvitol, starch, α-starch, dextrin, crystalline cellulose, low substitution hydroxypropyl cellulose, carboxymethyl cellulose sodium, gum arabic, pullulan, light anhydrous silicic acid, synthesized aluminum silicate, and magnesium aluminometasilicate.

Examples of the lubricant include magnesium stearate, calcium stearate, talc, and colloidal silica.

Examples of the binder include α-starch, sucrose, gelatin, gum arabic, methyl cellulose, carboxymethyl cellulose, carboxymethyl cellulose sodium, crystalline cellulose, sucrose, D-mannitol, trehalose, dextrin, pullulan, hydroxypropyl cellulose, hydroxypropyl methyl cellulose, and polyvinylpyrrolidone.

Examples of the disintegrating agent include lactose, white sugar, starch, carboxymethyl cellulose, carboxymethyl cellulose calcium, croscarmellose sodium, carboxymethyl starch sodium, light anhydrous silicic acid, and low substitution hydroxypropyl cellulose.

Examples of the solvent include an injectable solution, physiological saline water, Ringer solution, an alcohol, propylene glycol, polyethylene glycol, sesame oil, corn oil, olive oil, and cotton seed oil.

Examples of the solubilizer include polyethylene glycol, propylene glycol, D-mannitol, trehalose, benzyl benzoate, ethanol, trisaminomethane, cholesterol, triethanolamine, sodium carbonate, sodium citrate, sodium salicylate, and sodium acetate.

Examples of the suspending agent include surfactants such as stearyl triethanolamine, sodium lauryl sulfate, laurylaminopropionate, lecithin, benzalkonium chloride, benzethonium chloride, and glyceryl monostearate; hydrophilic polymers such as polyvinyl alcohol, polyvinylpyrrolidone, carboxymethyl cellulose sodium, methyl cellulose, hydroxymethyl cellulose, hydroxyethyl cellulose, and hydroxypropyl cellulose; polysorbates; and polyoxyethylene hydrogenated castor oil.

Examples of the isotonic agent include sodium chloride, glycerin, D-mannitol, D-solvitol, and glucose.

Examples of the buffer include buffer solutions of a phosphate salt, an acetate salt, a carbonate salt, and a citrate salt.

An example of the soothing agent includes benzyl alcohol.

Examples of the antiseptic agent include para-oxy benzoic acid esters, chlorobutanol, benzyl alcohol, phenethyl alcohol, dehydroacetic acid, and sorbic acid.

Examples of the antioxidant include a sulfate salt and an ascorbate salt.

Examples of the coloring agent include water-soluble food tar dyes (food dyes such as Food Red Nos. 2 and 3, Food Yellow Nos. 4 and 5, and Food Blue Nos. 1 and 2), water-insoluble lake dyes (aluminum salts of the above described water-soluble food tar dyes), and natural dyes (β-carotene, chlorophyll, bengala, etc.)

Examples of the sweetening agent include saccharin sodium, dipotassium glycyrrhizate, aspartame, and stevia.

The present compound or a pharmaceutically acceptable salt thereof can be used for inhibiting accumulation of Aβ as an active ingredient of an Aβ accumulation-inhibitory agent (hereinafter, may be referred to as the present Aβ accumulation-inhibitory agent).

Accumulation of Aβ can be inhibited by administering an effective amount of the present compound or a pharmaceutically acceptable salt thereof, or the present Aβ accumulation-inhibitory agent to a mammal which may be diagnosed with an Aβ-related disease.

For example, the present compound or a pharmaceutically acceptable salt thereof is used as the present Aβ accumulation-inhibitory agent, and its effective amount is orally or parenterally administered to a mammal such as a human, which may be diagnosed with an Aβ-related disease, so that the accumulation of Aβ can be inhibited in the mammal.

The present Aβ accumulation-inhibitory agent can be used as, for example, an oral agent such as a tablet (including a sublingual tablet and an oral disintegrating tablet), a capsule (including a soft capsule and a microcapsule), a granule, a powder, a lozenge, a syrup, an emulsion and a suspension, and a parenteral agent such as an injectable solution (e.g., a subcutaneous injectable solution, an intravenous injectable solution, an intramuscular injectable solution, an intraperitoneal injectable solution, and a drop), an external preparation (e.g., a percutaneous preparation and an ointment), a suppository (e.g., a rectal suppository and a vaginal suppository), a pellet, an intranasal agent, an intrapulmonary agent (e.g., a respiratory tonic), and an ophthalmic drop. In addition, these preparations (agents) may also be controlled-release system preparations such as a quick-release preparation or a sustained-release preparation (such as a sustained-release microcapsule).

An oral agent may be coated with a coating base, if necessary, for the purpose of masking of a taste, an enteric property or sustention.

Examples of the coating base include s sugar-coated base, a water-soluble film coating base, an enteric film coating base, and a sustained-release film coating base.

As the sugar-coated base, white sugar or the like is used, and further, one, or two or more selected from talc, precipitated calcium carbonate, gelatin, gum arabic, pullulan, carnauba wax, and the like may be used in combination.

Examples of the water-soluble film coating base include cellulose polymers such as hydroxypropyl cellulose, hydroxypropyl methyl cellulose, hydroxyethyl cellulose, and methyl hydroxyethyl cellulose; synthesized polymers such as polyvinylacetal diethylaminoacetate, aminoalkyl methacrylate copolymer E, and polyvinylpyrrolidone; and polysaccharide such as pullulan

Examples of the enteric film coating base include cellulose polymers such as hydroxypropyl methyl cellulose phthalate, hydroxypropyl methyl cellulose acetate succinate, carboxymethyl ethyl cellulose, and cellulose acetate phthalate; acrylic acid polymers such as methacrylic acid copolymer L, methacrylic acid copolymer LD, and methacrylic acid copolymer S; and natural products such as shellac.

Examples of the sustained-release film coating base include cellulose polymers such as ethyl cellulose; and acrylic acid polymers such as aminoalkyl methacrylate copolymer RS, and an ethyl acrylate-methyl methacrylate copolymer suspension.

The above described coating bases may be used in mixing two or more kinds thereof in a suitable ratio. In coating, for example, a light blocking agent such as titanium oxide or iron sesquioxide may also be used.

The present Aβ accumulation-inhibitory agent can be produced by a conventional method in the field of pharmaceutical preparation technology, for example, methods described in Japanese Pharmacopoeia.

The content of the present compound in the present Aβ accumulation-inhibitory agent is, for example, about 0.1 to 100% by weight, although it depends on a dosage form, an absorption efficiency of the compound, and so on.

The present Aβ accumulation-inhibitory agent may further contain, for example, a pharmaceutically acceptable carrier, etc. in addition to the present compound or a pharmaceutically acceptable salt thereof. Such a pharmaceutically acceptable carrier, etc. are the same as the carrier and the like used in the above described present pharmaceutical composition.

As the present Aβ accumulation-inhibitory agent thus obtained is low-toxic, and thus highly safe, it may be used (administered) to, for example, a mammal (for example, a human, a rat, a mouse, a guinea pig, a rabbit, a sheep, a pig, a cow, a horse, a cat, a dog and a monkey). The dosage varies depending on age, sex, weight and severity of a disease of a mammal to be administered, a kind and an administration form (dosage form) of the present Aβ accumulation-inhibitory agent, and so on. Usually, for example, in the case of orally administering to a human, it may be administered at, as the amount of an active ingredient, 0.01 mg to 1000 mg, preferably 0.1 mg to 1000 mg, more preferably 1.0 mg to 200 mg, and even more preferably 1.0 mg to 50 mg per day, to an adult (for example, weighing 60 kg). The daily dose may also be administered in several divided dosage.

Also, in the case of parenterally administering to a human, it may be administered by an intravenous injection at, as the amount of an active ingredient, 0.01 mg to 30 mg, preferably 0.1 mg to 20 mg, and more preferably 1.0 mg to 10 mg per day, to an adult (for example, weighing 60 kg).

Administration method of the present Aβ accumulation-inhibitory agent is not limited to oral or parenteral administration, and those may be used singly or in combination.

Examples of a disease to which the present Aβ accumulation-inhibitory agent may be applicable include such a disease as an Aβ-related disease (amyloidosis).

The Aβ-related disease is classified into "localized amyloidosis" in which accumulation (deposition) is caused in only some organs and tissues in a living body, and "systemic amyloidosis" in which accumulation is caused in various organs and tissues throughout the body.

Examples of localized amyloidosis include Alzheimer's disease, Down's syndrome, cerebrovascular amyloidosis, hereditary amyloidotic cerebral hemorrhage, mad cow disease (bovine spongiform encephalopathy, BSE), variant Creutzfeldt-Jakob disease (vCJD), Gerstmann-Straussler-Scheinker syndrome, transmissible spongiform encephalopathy, scrapie, heart disease (senile cardiac amyloidosis), endocrine tumor (thyroid cancer), adult-onset diabetes, cutaneous amyloidosis and localized nodular amyloidosis.

Examples of systemic amyloidosis include familial amyloid polyneuropathy (FAP), tuberculosis, Hansen's disease, familial Mediterranean fever, bronchiectasis, Muckle-wells syndrome, reactive AA amyloidosis, immunocytic amyloidosis, osteomyelitis and cardiac failure. Other examples include senile amyloidosis which develops non-hereditarily at an advanced age, dialysis amyloidosis caused by amyloid that results from alteration in protein irremovable by a dialysis membrane used in treatment of dialysis patients, and secondary amyloidosis caused by amyloid that is generated by cleavage of a protein expressed in rheumatism.

### Examples

The present invention will be described below in further detail by example, however, the present invention is not limited by it.

### Example 1 Measurement of fluctuation in the amount of Aβ accumulation in cultured nerve cells

Using a phenol red-free DMEM medium (manufactured by Invitrogen Corporation) to which charcoal dextran-treated FBS has been added so that its final concentration can be 10%, cultured nerve cells (human neuroblastoma CHP212, ATCC) were inoculated in a six-well plate (manufactured by BD Falcon) at approximately 2 x 10⁵ cells/well, and then cultured overnight. After the cultivation, DMSO or a test substance of the formula (II) dissolved in DMSO was added to the cells. The cells were then cultured, and 48 hours after the addition of the test substance, a protease inhibitor (p1860, manufactured by Sigma-Aldrich Corporation) was added to each well so that its final concentration can be 1:100, followed by measuring the amount of Aβ in the medium by ELISA method (Human/Rat Amyloid β1-42 measuring kit, Code. No. 290-62601, Wako Pure Chemical Industries, Ltd.) The results were shown in Figure 1.

It was observed that the amount of Aβ accumulation was markedly decreased in a culture that had been treated with the test substance of the formula (II).

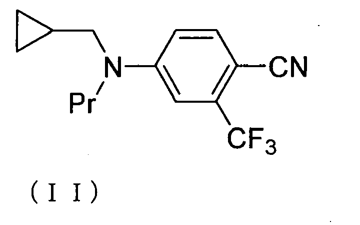

### Industrial Applicability

According to the present invention, it becomes possible to provide a compound that inhibits Aβ accumulation.

## Claims

1. A pharmaceutical composition for inhibiting amyloid-β protein accumulation comprising a compound of formula (I): 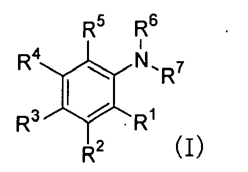 or a pharmaceutically acceptable salt thereof,
in the formula,
R¹ is H, cyano, nitro, halogen, C1-C12 haloalkyl, C1-C12 alkyl, C2-C12 alkenyl, C2-C12 alkynyl, hydroxy, C1-C12 alkoxy, C1-C12 haloalkoxy, -OC(O)R⁸ or aryl;
R² is H, cyano, nitro, halogen, C1-C12 haloalkyl, C1-C12 alkyl, C2-C12 alkenyl, C2-C12 alkynyl, hydroxy, C1-C12 alkoxy, C1-C12 haloalkoxy, -OC(O)R⁸ or aryl;
R³ is cyano, nitro, halogen, C1-C12 haloalkyl, C1-C12 heterocyclyl, hydroxy, C1-C12 alkoxy, C1-C12 haloalkoxy, -OC(O)R⁸, -C(O)OR^{8'}, -CONHR^{8'}, -C(O)R⁸, -S(O)ₙR^{8'}, -SO₂NR^{8'}R^{8"}, -NHC(O)R⁸ or -NHSO₂R⁸ ,wherein n is 0, 1 or 2;
R⁴ is H, cyano, nitro, halogen, C1-C12 haloalkyl, C1-C12 alkyl, C2-C12 alkenyl, C2-C12 alkynyl, hydroxy, C1-C12 alkoxy, C1-C12 haloalkoxy, -OC(O)R⁸ or aryl;
R⁵ is H, cyano, nitro, halogen, C1-C12 haloalkyl, C1-C12 alkyl, C2-C12 alkenyl, C2-C12 alkynyl, hydroxy, C1-C12 alkoxy, C1-C12 haloalkoxy, -OC(O)R⁸ or aryl;
herein, at least one of R¹ , R², R⁴ and R⁵ is not H;
each of R⁶ and R⁷ independently are selected from H or -(R^{a})x-R⁹ ;
R^{a} is a C₁-C₈alkylene chain, where x is 0 or 1;
each R⁸ independently is H, C1-C12 alkyl, C2-C12 alkenyl, C2-C12 alkynyl, C3-C12 cycloalkyl, C2-C12 heterocyclyl, aryl, aryl(C1-C12)alkyl, heteroaryl or heteroaryl(C1-C12)alkyl;
R⁸' a n d R⁸" each independently are H, C1-C12 alkyl, C3-C12 alkenyl, C3-C12 alkynyl, C3-C12 cycloalkyl, C3-C12 heterocyclyl, aryl, aryl(C1-C12)alkyl, heteroaryl or heteroaryl(C1-C12)alkyl;
R⁹ is C1-C12 alkyl, C1-C12 haloalkyl, C2-C12 hydroxyalkyl, C3-C12 alkenyl, C3-C12 alkynyl, C1-C12 alkoxy, C1-C12 alkylthio, C2-C12 haloalkoxy, C3-C12 cycloalkyl, formyl, azide or -NR¹⁰R¹¹;
R¹⁰ and R¹¹ each independently are H, C1-C12 alkyl, -C(O)H, -C(O)R¹², -C(O)OR¹² or -SO₂R¹²; and
R¹² is C1-C12 alkyl;
provided that when R⁶ is methoxy or methylthio, R⁷ simultaneously is not methoxy or methylthio, and when R⁹ is trifluoromethyl or azide, x is not 0, and when R⁹ is azide, R^{a} is not methylene.

2. An amyloid-β protein accumulation-inhibitory agent for inhibiting amyloid-β protein accumulation comprising the compound of formula (I) described in Claim 1 or a pharmaceutically acceptable salt thereof.

3. Use of the compound of formula (I) described in Claim 1 or a pharmaceutically acceptable salt thereof for inhibiting amyloid-β protein accumulation.

4. A method for inhibiting amyloid-β protein accumulation, comprising a step of administering an effective amount of the compound of formula (I) described in Claim 1 or a pharmaceutically acceptable salt thereof to a mammal which may be diagnosed with an amyloid-β protein-related disease.

5. A pharmaceutical composition for inhibiting amyloid-β protein accumulation comprising the compound of formula (I) described in Claim 1 or a pharmaceutically acceptable salt thereof, wherein at least one of R¹ and R⁵ is H.

6. A pharmaceutical composition for inhibiting amyloid-β protein accumulation comprising the compound of formula (I) described in Claim 1 or a pharmaceutically acceptable salt thereof, wherein at least one of R² and R⁴ is H.

7. A pharmaceutical composition for inhibiting amyloid-β protein accumulation comprising the compound of formula (I) described in Claim 1 or a pharmaceutically acceptable salt thereof, wherein C1-C12 alkyl is C1-C6 alkyl.

8. A pharmaceutical composition for inhibiting amyloid-β protein accumulation comprising the compound of formula (I) described in Claim 1 or a pharmaceutically acceptable salt thereof, wherein C1-C12 alkoxy is C1-C6 alkoxy.

9. A pharmaceutical composition for inhibiting amyloid-β protein accumulation comprising the compound of formula (I) described in Claim 1 or a pharmaceutically acceptable salt thereof, wherein C1-C12 haloalkyl is C1-C6 haloalkyl.

10. A pharmaceutical composition for inhibiting amyloid-β protein accumulation comprising the compound of formula (I) described in Claim 1 or a pharmaceutically acceptable salt thereof, wherein C2-C12 alkenyl of R¹, R², R⁴, R⁵ and R⁸ is C2-C6 alkenyl, and wherein C3-C12 alkenyl of R^{8'}, R⁸" and R⁹ is C3-C6 alkenyl.

11. A pharmaceutical composition for inhibiting amyloid-β protein accumulation comprisinig the compound of formula (I) described in Claim 1 or a pharmaceutically acceptable salt thereof, wherein C2-C12 alkynyl of R¹, R², R⁴ , R⁵ and R⁸ is C2-C6 alkynyl, and wherein C3-C12 alkynyl of R^{8'}, R^{8"} and R⁹ is C3-C6 alkynyl.

12. A pharmaceutical composition for inhibiting amyloid-β protein accumulation comprising the compound of formula (I) described in Claim 1 or a pharmaceutically acceptable salt thereof, wherein C3-C12 cycloalkyl is C3-C6 cycloalkyl.

13. A pharmaceutical composition for inhibiting amyloid-β protein accumulation comprising the compound of the formula (I) described in Claim 1 or a pharmaceutically acceptable salt thereof, wherein R¹ or R⁵ is nitro, C1-C6 alkyl, C1-C6 haloalkyl or halogen.

14. A pharmaceutical composition for inhibiting amyloid-β protein accumulation comprising the compound of formula (I) described in Claim 1 or a pharmaceutically acceptable salt thereof, wherein R² or R⁴ is nitro, cyano, C1-C6 alkyl, C1-C6 haloalkyl, halogen or hydroxy.

15. A pharmaceutical composition for inhibiting amyloid-β protein accumulation comprising the compound of formula (I) described in Claim 1 or a pharmaceutically acceptable salt thereof, wherein R³ is nitro, cyano or halogen.

16. A pharmaceutical composition for inhibiting amyloid-β protein accumulation comprising the compound of formula (I) described in Claim 1 or a pharmaceutically acceptable salt thereof, wherein R⁹ is C1-C6 alkyl, C3-C6 alkenyl or C2-C6 hydroxyalkyl.
